# EUROPEAN PATENT APPLICATION

(11) **EP 4 666 865 A2**
(43) Date of publication of application: **24.12.2025**
(21) Application number: 25213263.4
(22) Date of filing: 01.03.2019
(51) Int. Cl.: A23K 20/158, A23K 50/30

(54) **CHEMICAL MITIGATION OF AFRICAN SWINE FEVER VIRUS AND CLASSICAL SWINE FEVER VIRUS**

(30) Priority: 02.03.2018 US 201862637825 P; 17.12.2018 US 201862780740 P
(62) Divisional of application: 19760046.3
(71) Applicant: Kansas State University Research Foundation, Manhattan, KS 66502 (US)
(72) Inventor: NIEDERWERDER, Megan C., Manhattan, 66506 (US); ROWLAND, Raymond R.R, Manhattan, 66502 (US); JONES, Cassandra, Wamego, 66547 (US); DRITZ, Steven S., Manhattan, 66503 (US); WOODWORTH, Jason C, Enterprise, 66503 (US)
(74) Representative: Murgitroyd & Company

(57) **Abstract**

Methods of inhibiting the spread of African swine fever virus and/or classical swine fever virus in animal feed, feed ingredients, and pet food are provided. The methods utilize generally safe chemical mitigants, such as medium chain fatty acids. The chemical mitigants are effective when introduced to the feed or feed ingredients at inclusion rates much lower than previous methods for inhibiting other microbes. The methods are particularly suitable for use in post-processing treatment of animal feed, feed ingredients, or pet food that will be transported and stored for multiple days or weeks.

## Description

### CROSS-REFERENCE TO RELATED APPLICATIONS

The present application claims the priority benefit of U.S. Provisional Patent Application Serial No. 62/637,825, filed March 2, 2018, entitled INACTIVATION OF VIRUSES SUCH AS AFRICAN SWINE FEVER VIRUS (ASFV) AND CLASSICAL SWINE FEVER VIRUS (CSFV) WITH MEDIUM CHAIN FATTY ACIDS, and U.S. Provisional Patent Application Serial No. 62/780,740, filed December 17, 2018, entitled CHEMICAL MITIGATION OF AFRICAN SWINE FEVER VIRUS, each of which are incorporated by reference in their entireties herein.

### BACKGROUND OF THE INVENTION

### Field of the Invention

The present invention is broadly concerned with methods of inhibiting African Swine Fever Virus and/or Classical Swine Fever Virus in animal feed, feed ingredients, and pet food.

### Description of Related Art

Medium chain fatty acids have been shown to be effective against specific domestic pathogens, porcine epidemic diarrhea virus (PEDV) and *Salmonella sp.* (U.S. Patent Application Publication No. 2017/0354167, published December 14, 2017, incorporated by reference herein in its entirety).

African swine fever virus and classical swine fever virus are foreign animal diseases known to be transmitted through the oral route. In particular, African swine fever virus (ASFV) is a very large complex DNA virus that is rapidly spreading through the largest pork producing country in the world, China. ASFV causes high mortality in pigs and is currently a foreign animal disease to North America and most European countries. There is currently no effective vaccine and the virus is known to be transmitted through the oral route. ASFV is capable of surviving in feed and feed ingredients subjected to varying environmental conditions simulating transoceanic shipment. ASFV is a very unique double-stranded DNA virus and is the only virus in the family *Asfarviridae* and genus *Asfivirus.* Importantly, there are no appropriate surrogate viruses for ASFV. Classical swine fever virus (CSFV) is a single-stranded RNA virus in the *Flaviviridae* family. Porcine epidemic diarrhea virus (PEDV) is unrelated to either virus and is in the family *Coronaviridae.* The genomes of these three viruses are very different in size, with PEDV being 28 kb compared to 12.3 kb for CSFV and 190 kb for ASFV. It is well known that viruses vary tremendously with regards to stability in the environment and sensitivity to disinfectants. Even viruses within the same family can have different characteristics with regards to inactivation rates, and none of these three viruses are in the same family. A published study has compared stability between several viruses causing foreign animal diseases demonstrating that significant variation exists. Additionally, other previously published work demonstrates significant variation in terms of stability in feed ingredients between PEDV, ASFV and bovine viral diarrhea virus (BVDV, a virus in the same family and used as a surrogate for CSFV). Variability in terms of stability in feed ingredients would predict differences in terms of sensitivity to MCFA and mitigation. Thus, the effects of mitigants on other viruses or bacteria cannot be extended or translated to ASFV or CSFV without direct evidence of the mitigant on the virus itself.

What is needed are treatments that are effective at mitigating (e.g., inactivating) ASFV and CSFV in animal feed and feed ingredients, while also being safe for oral administration to pigs and other animals.

### SUMMARY OF THE INVENTION

The current application describes methods for inhibiting African swine fever virus and/or classical swine fever virus in animal feed or animal feed ingredients. The methods comprise introducing a chemical mitigant to the feed or feed ingredients. The chemical mitigant comprises (consists essentially or even consists of) a medium chain fatty acid and/or an essential oil, and the chemical mitigant is introduced to the feed or feed ingredients at an inclusion rate of less than 2 weight % (but generally at least about 0.125 weight %), based upon the total weight of the animal feed or feed ingredient taken as 100% by weight.

In another embodiment, there is provided chemical mitigants for use in inhibiting African swine fever virus and/or classical swine fever virus in animal feed or animal feed ingredients. The chemical mitigants comprise a medium chain fatty acid and/or an essential oil.

Also described herein are treated animal feeds and/or animal feed ingredients having resistance to African swine fever virus and/or classical swine fever virus. The feed or feed ingredients comprise from about 0.125 weight % to less than 2 weight % of a chemical mitigant, based upon the total weight of the animal feed or feed ingredient taken as 100% by weight. The chemical mitigants comprise a medium chain fatty acid and/or an essential oil. Exemplary treated animal feed or animal feed ingredients for use in the invention include complete swine diet, blood meal, porcine meat and bone meal (MBM), spray-dried animal plasma, feather meal, avian blood meal, poultry by-product meal, vitamin D, lysine hydrochloride, choline chloride, soybean meal, dry pet kibble, and mixtures thereof.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
Figure (Fig.) 1 is a graph showing the dose response inactivation curve of ASFV (strain BA71V) exposed to varying concentrations of a 1:1:1 MCFA blend, with data shown as the titer after exposure to MCFA concentrations between 0.125% and 2.0% along with the percent reduction of virus concentration compared to the positive control;
Fig. 2 is a series of graphs showing detection of ASFV Georgia 2007 genome over the course of the 30-day transboundary model, with data shown as the mean cycle threshold (Ct) values for duplicate replicates at days 1, 8, 17 and 30 post-inoculation; and
Fig. 3 is a graph showing quantity of ASFV DNA as measured by qPCR at the conclusion of the 30-day transboundary model in nontreated controls (open bars) and samples treated with MCFA at 28 dpi (black bars);
Fig. 4 is a graph showing the dose response inactivation curve of CSFV (Brescia isolate) exposed to varying concentrations of a 1:1:1 MCFA C6:C8:C10 blend, with the data shown as the titer after exposure to MCFA concentrations between 0.125% and 2.0% along with the percent reduction of virus concentration compared to the positive control; and
Fig. 5A is the positive control image from experiments on indirect fluorescent antibody detection of CSFV Brescia on porcine kidney cells with and without exposure to MCFA;
Fig. 5B is an image from experiments on indirect fluorescent antibody detection of CSFV Brescia on porcine kidney cells treated with 0.625% MCFA; and
Fig. 5C is the negative control image from experiments on indirect fluorescent antibody detection of CSFV Brescia on porcine kidney cells with and without exposure to MCFA.

### DETAILED DESCRIPTION OF THE PREFERRED EMBODIMENTS

The present invention is generally concerned with methods of inhibiting African swine fever virus (ASFV) and/or classical swine fever virus (CSFV) in animal feed, feed ingredients, and pet food. More specifically, the present invention relates to chemical mitigants for use in inhibiting ASFV and/or CSFV in various types of animal and pet food ingredients, as well as complete feed meals and pet food products. In general, the chemical mitigants comprise a medium chain fatty acid and/or an essential oil. As used herein, "inhibit" or "inhibiting" refers to the reduction of the measurable levels of the target microbe (i.e., ASFV or CSFV) or decrease in the rate of growth of the microbe as compared to an untreated control. In one or more embodiments, methods in accordance with the present invention use an effective amount of a chemical mitigant to inhibit ASFV and/or CSFV in animal feed or animal feed ingredients, for example, to concentrations below the levels of detection through RT-PCR and/or virus isolation in cell culture. As used herein, an "effective amount" refers to an amount capable of providing bioavailable levels of the active compound (e.g., medium chain fatty acids and/or essential oils) sufficient to achieve the desired performance improvement. In preferred embodiments, methods in accordance with the present invention are advantageously adapted to be used in animal feed ingredient transport and storage.

Chemical mitigants for use in one or more embodiments of the present invention can include medium chain fatty acids and/or essential oils. In one or more embodiments, the chemical mitigant comprises (consists essentially or even consists of) medium chain fatty acids, and more specifically at least one medium chain fatty acid. Medium chain fatty acids are acids having an aliphatic tail of 6 to 12 carbon atoms. In one or more embodiments, medium chain fatty acids for use in the present invention include caproic acid, caprylic acid, capric acid, and/or lauric acid. Therefore, in certain embodiments, the chemical mitigant is selected from the group consisting of caproic acid, caprylic acid, capric acid, lauric acid, and mixtures thereof. However, in certain other embodiments, the chemical mitigant is free of lauric acid. Therefore, in such embodiments the chemical mitigant may be selected from the group consisting of caproic acid, caprylic acid, capric acid, and mixtures thereof. In one or more embodiments, a blend of medium chain fatty acids may be used. For example, in one or more embodiments, a blend of two or more medium chain fatty acids may be introduced to the feed or feed ingredients. In one or more embodiments, a blend of caproic acid, caprylic acid, and capric acid is introduced to the feed or feed ingredients at a weight ratio of about 1:1:1 (equal parts). This combination increases solubility of the medium chain fatty acids and is effective in inactivating viruses as well as improving growth of pigs when administered orally. However, it is within the scope of the present invention that blends comprising other weight ratios of caproic acid, caprylic acid, and capric acid may be used.

In one or more embodiments, the chemical mitigant comprises (consists essentially or even consists of) essential oils, and more specifically at least one essential oil. Essential oils are concentrated hydrophobic liquids containing volatile aromatic compounds derived from plants. A number of different essential oils exist which may be used in one or more embodiments of the present invention. A non-exclusive list of these essential oils include: Agar oil, Ajwain oil, Angelica root oil, Anise oil, Asafoetida, Balsam of Peru, Basil oil, Bay oil, Bergamot oil, Black Pepper, Buchu oil, Birch, Camphor, Cannabis flower essential oil, Caraway oil, Cardamom seed oil, Carrot seed oil, Cedarwood oil, Chamomile oil, Calamus Root, Cinnamon oil, Cistus species, Citron, Citronella oil, Clary Sage, Clove oil, Coffee, Coriander, Costmary oil (bible leaf oil), Costus Root, Cranberry seed oil, Cubeb, Cumin oil/Black seed oil, Cypress, Cypriol, Curry leaf, Davana oil, Dill oil, Elecampane, Eucalyptus oil, Fennel seed oil, Fenugreek oil, Fir, Frankincense oil, Galangal, Galbanum, Geranium oil, Ginger oil, Goldenrod, Grapefruit oil, Henna oil, Helichrysum, Hickory nut oil, Horseradish oil, Hyssop, Idaho Tansy, Jasmine oil, Juniper berry oil, Laurus nobilis, Lavender oil, Ledum, Lemon oil, Lemongrass, Lime, Litsea cubeba oil, Linaloe, Mandarin, Marjoram, Melaleuca See Tea tree oil, Melissa oil (Lemon balm), Mentha arvensis oil/Mint oil, Moringa oil, Mountain Savory, Mugwort oil, Mustard oil (essential oil), Myrrh oil, Myrtle, Neem oil or Neem Tree Oil, Neroli, Nutmeg, Orange oil, Oregano oil, Orris oil, Palo Santo, Parsley oil, Patchouli oil, Perilla essential oil, Pennyroyal oil, Peppermint oil, Petitgrain, Pine oil, Ravensara, Red Cedar, Roman Chamomile, Rose oil, Rosehip oil, Rosemary oil, Rosewood oil, Sage oil, Sandalwood oil, Sassafras oil, Savory oil, Schisandra oil, Spearmint oil, Spikenard, Spruce, Star anise oil, Tangerine, Tarragon oil, Tea tree oil, Thyme oil, Tsuga, Turmeric, Valerian, Vetiver oil (khus oil), Western red cedar, Wintergreen, Yarrow oil, Ylang-ylang, and Zedoary. In one or more embodiments, the chemical mitigant is selected from the group consisting of garlic oleoresin, turmeric oleoresin, capsicum oleoresin, rosemary extract, wild oregano essential oil, and mixtures thereof. In one or more embodiments, a blend of essential oils may be used. For example, in one or more embodiments, a blend of two or more essential oils may be introduced to the feed or feed ingredients. In one or more embodiments, a blend of essential oils comprising equal parts of garlic oleoresin, turmeric oleoresin, capsicum oleoresin, rosemary extract, and wild oregano essential oil is introduced to the feed or feed ingredients. However, it is within the scope of the present invention that blends comprising other weight ratios of garlic oleoresin, turmeric oleoresin, capsicum oleoresin, rosemary extract, and wild oregano essential oil may be used.

In one or more embodiments, a blend comprising (consisting essentially or even consisting of) medium chain fatty acids and essential oils, and more specifically one or more medium chain fatty acids and one or more essential oils, may be used as the chemical mitigant. However, in certain other embodiments, no essential oils or other types of mitigants are used, except for medium chain fatty acids. Additionally, embodiments of the present invention avoid the use of toxic chemicals, such as formaldehyde, which are not introduced to the animal feed, feed ingredients, or pet food. Thus, in one or more embodiments, the chemical mitigant consists essentially (or even consists) of one or more medium chain fatty acids. In certain preferred embodiments, the chemical mitigant consists essentially (or even consists) of a blend of caproic acid, caprylic acid, and capric acid.

While any effective amount of chemical mitigant may be used, in one or more embodiments the chemical mitigant is introduced to the animal feed (including pet food) or feed ingredients at an inclusion rate of from about 0.01 weight % to about 10 weight %, preferably from about 0.05 weight % to about 5 weight %, more preferably from about 0.1 weight % to about 2 weight %, and most preferably about 0.5 weight % to about 0.9 weight %, based upon the total weight of the feed or feed ingredient taken as 100% by weight. Advantageously, the chemical mitigants described herein, and particularly medium chain fatty acids, are effective inactivants (i.e., 4-log reduction) of ASFV and CSFV at inclusion rates as low as 0.6 weight % (for ASFV) and 0.5% weight % (for CSFV), much lower than doses shown to be effective in the prior art for other microbes. Thus, in one or more embodiments the chemical mitigant is introduced to the animal feed or feed ingredients at an inclusion rate of less than 2 weight %, less than 1.5 weight %, less than 1 weight %, less than 0.9 weight %, less than 0.8 weight %, less than 0.7 weight %, or less than 0.6 weight %, based upon the total weight of the feed or feed ingredient taken as 100% by weight. In one or more embodiments, lower doses may also be used, for example when 4-log reduction of ASFV or CSFV is not necessary. In one or more such embodiments, the chemical mitigant may introduced to the animal feed (including pet food) or feed ingredients at an inclusion rate of from about 0.125 weight % to about 0.5 weight %, based upon the total weight of the feed or feed ingredient taken as 100% by weight. However, in certain other embodiments, the chemical mitigant is introduced to the animal feed or feed ingredients at an inclusion rate of at least 0.125 weight %, at least 0.25 weight %, at least 0.5 weight %, at least 0.6 weight %, at least 0.7 weight %, at least 0.8 weight %, at least 0.9 weight %, or at least 1 weight %, based upon the total weight of the feed or feed ingredient taken as 100% by weight.

In one or more embodiments, sodium bisulfate may also be added to the animal feed or feed ingredients, in addition to the medium chain fatty acids and/or essential oils. Sodium bisulfate is an acid salt that is considered "Generally Recognized as Safe" (GRAS) and a "natural product" by the FDA. In one or more embodiments, sodium bisulfate can be dissolved into solution and applied to the surface of an animal food or food ingredient to prevent or decrease bacteria growth. In one or more embodiments, sodium bisulfate solution is applied to the surface of a dry pet food or pet food ingredient. For example, the solution may be applied to the surface of dry dog food (kibbles) or dry cat food. In one or more embodiments the solution may be applied to the surface of the feed or ingredient so as to provide sodium bisulfate at an inclusion rate of from about 0.1 weight % to about 2 weight %, more preferably from about 0.15 weight % to about 1.5 weight %, even more preferably from about 0.2 weight % to about 1 weight %, based upon the total weight of the feed or feed ingredient taken as 100% by weight.

Chemical mitigants used in accordance with the present invention may be used to treat a wide variety of animal feed or animal feed ingredients. In one or more embodiments, however, the methods in accordance with the present invention are particularly suited for use with porcine feed and feed ingredients. In such embodiments, the animal feed or animal feed ingredients may be selected from the group consisting of complete swine diet, blood meal, porcine meat and bone meal (MBM), and spray-dried animal plasma. In one or more embodiments, the animal feed ingredients may comprise ingredients selected from the group consisting of vitamin D, lysine hydrochloride, choline chloride, and soybean meal. In other embodiments, the chemical mitigant may be used with pet food and pet food ingredients. In one or more embodiments, the pet food and pet food ingredients comprise dry dog food (kibble) and/or cat food. The term "pet food" means any composition intended to be consumed by a pet, and "dry" food refers generally in the art to pet food having a moisture content of about less than 20% (preferably less than about 15%, more preferably less than about 10%). The term "kibble" is used in the art to refer to pellets of dry pet food.

Methods in accordance with one or more embodiments of the present invention may be used in the production of animal or pet feeds. Therefore, in one embodiment of the present invention, there is provided an animal feed or pet food comprising from about 0.01 weight % to about 10 weight %, more preferably from about 0.05 weight % to about 5 weight %, even more preferably from about 0.1 weight % to about 2 weight %, and most preferably about 0.5 weight % to about 0.9 weight %, of a chemical mitigant or blend of chemical mitigants (such as the chemical mitigants and blends described herein), based upon the total weight of the feed taken as 100% by weight. In one or more embodiments, there is provided an animal feed or pet food comprising less than 2 weight %, less than 1.5 weight %, less than 1 weight %, less than 0.9 weight%, less than 0.8 weight %, less than 0.7 weight %, or less than 0.6 weight %, of a chemical mitigant or blend of chemical mitigants (such as the chemical mitigants and blends described herein), based upon the total weight of the feed taken as 100% by weight. In one or more embodiments, there is provided an animal feed or pet food comprising from about 0.125 weight % to about 0.5 weight %, of a chemical mitigant or blend of chemical mitigants (such as the chemical mitigants and blends described herein), based upon the total weight of the feed taken as 100% by weight. In one or more embodiments, there is provided an animal feed or pet food comprising at least 0.5 weight %, at least 0.6 weight %, at least 0.7 weight %, at least 0.8 weight %, at least 0.9 weight %, or at least 1 weight %, of a chemical mitigant or blend of chemical mitigants (such as the chemical mitigants and blends described herein), based upon the total weight of the feed taken as 100% by weight.

Embodiments of the present invention are particularly suitable for use in the transport of feed and feed ingredients, especially international transport and storage. Prior to the present invention, medium chain fatty acids had not been shown as an effective mitigant against diseases, and especially viruses, foreign to the United States industry. Feed or ingredients may become contaminated with ASFV and/or CSFV at the point of processing. Advantageously, embodiments of the present invention are particularly suitable to inactivate and inhibit the spread of these contaminants. Therefore, in one or more embodiments, methods in accordance with the present invention comprise introducing the chemical mitigant to the feed or feed ingredients after processing. The chemical mitigant may be mixed with the feed or feed ingredients for sufficient time so as to provide a homogeneous mixture. In one or more embodiments, methods in accordance with the present invention may prevent or decrease ASFV and/or CSFV in feed and/or ingredients for at least about 90 days of transport and storage after processing, at least about 60 days of transport and storage after processing, at least about 40 days of transport and storage after processing, or at least about 30 days of transport and storage after processing.

Embodiments of the present invention advantageously provide a safe alternative method of preventing or decreasing ASFV and/or CSFV in animal or pet feed and ingredients. Prior methods using harmful chemicals have displayed negative effects on protein and amino acid metabolism of animals. Unlike prior methods, the present invention uses generally non-hazardous chemical mitigants at doses discovered to achieve effective mitigation of ASFV and/or CSFV. The chemical mitigants used in accordance with the present invention are natural alternatives that pose essentially no risk to the safety of workers or the environment.

Additional advantages of the various embodiments of the invention will be apparent to those skilled in the art upon review of the disclosure herein and the working examples below. It will be appreciated that the various embodiments described herein are not necessarily mutually exclusive unless otherwise indicated herein. For example, a feature described or depicted in one embodiment may also be included in other embodiments, but is not necessarily included. Thus, the present invention encompasses a variety of combinations and/or integrations of the specific embodiments described herein.

As used herein, the phrase "and/or," when used in a list of two or more items, means that any one of the listed items can be employed by itself or any combination of two or more of the listed items can be employed. For example, if a composition is described as containing or excluding components A, B, and/or C, the composition can contain or exclude A alone; B alone; C alone; A and B in combination; A and C in combination; B and C in combination; or A, B, and C in combination.

The present description also uses numerical ranges to quantify certain parameters relating to various embodiments of the invention. It should be understood that when numerical ranges are provided, such ranges are to be construed as providing literal support for claim limitations that only recite the lower value of the range as well as claim limitations that only recite the upper value of the range. For example, a disclosed numerical range of about 10 to about 100 provides literal support for a claim reciting "greater than or equal to about 10" (with no upper bounds) and a claim reciting "less than or equal to about 100" (with no lower bounds).

### EXAMPLES

The following examples set forth the effectiveness of chemical mitigation strategies on ASFV and CSFV in feed and feed ingredients. It is to be understood, however, that these examples are provided by way of illustration and nothing therein should be taken as a limitation upon the overall scope of the invention.

Protocols and procedures were developed for diluting and mixing various concentrations of a medium chain fatty acid blend (MCFA) with ASFV (BA71v isolate) and CSFV (Brescia isolate). This work has been performed in their respective cell cultures, vero cells and porcine kidney cells. As a first step, the MCFA is prepared by mixing equal volumes of C6:C8:C10 (caproic acid : caprylic acid : capric acid) for a 1:1:1 volume ratio. Second, MCFA treatments are prepared at concentrations ranging between 10% and 0.625% and mixed with a standard high concentration of virus (10⁶ TCID₅₀/ml). It has been confirmed that MCFA does not disrupt the cell cultures used for these experiments and have included positive and negative controls in each assay. Preliminary results demonstrated that MCFA effectively inactivates both CSFV and ASFV at all doses tested between 10% and 0.625%.

### EXAMPLE I

### African swine fever virus testing

Specific protocols and procedures were used for diluting and mixing various concentrations of a medium chain fatty acids blend in 20% DMSO with ASFV (BA71v isolate) in vero cells. As a first step, the medium chain fatty acids were prepared by mixing equal volumes of C6:C8:C10 for a 1:1:1 volume ratio in 20% DMSO. Second, medium chain fatty acids were prepared at concentrations ranging between 2% and 0.125% and mixed with a standard high concentration of ASFV (10⁶ TCID₅₀/ml). Positive controls were included in each assay to determine the dose response inactivation of the virus.

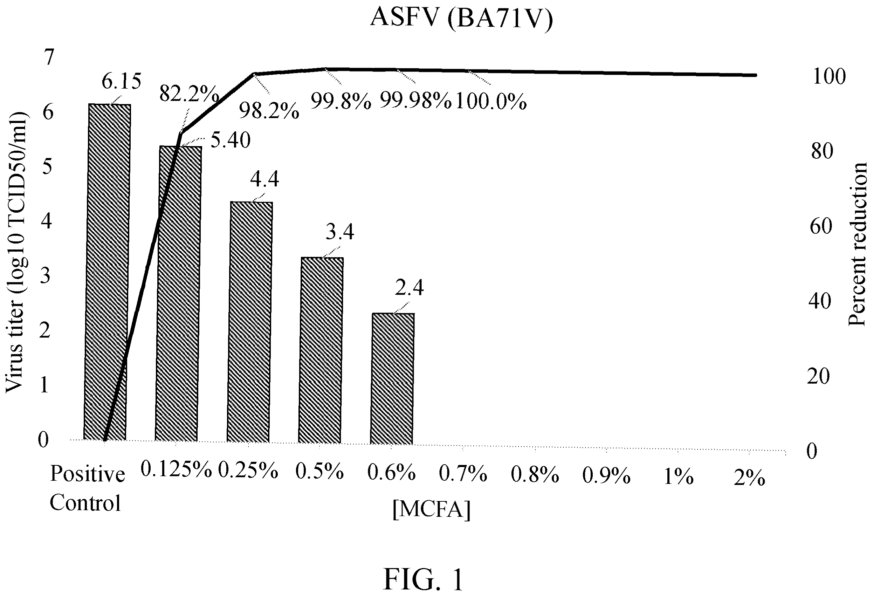

Results (Table 1, Fig. 1) demonstrated that medium chain fatty acids treatments effectively inactivate ASFV to undetectable levels by indirect fluorescent antibody testing at all doses tested between 0.7% and 2.0%. Dose dependent reduction of ASFV is seen at medium chain fatty acids concentrations between 0.6% and 0.125%. At the lowest concentration of medium chain fatty acids tested in cell culture (0.125% medium chain fatty acids), there is an approximate 0.75 log₁₀ TCID₅₀/ml reduction in virus titer. At 0.25% medium chain fatty acids inclusion, virus titers are reduced by approximately 98.2%. An approximate 4-log reduction in virus titer is seen at 0.6% medium chain fatty acids inclusion. A 4-log reduction in virus titer is the standard described by the World Organization for Animal Health (OIE) for virus inactivation. In summary, we have demonstrated that medium chain fatty acids are an effective inactivant of ASFV in cell culture and that the dose required for an approximate 4-log reduction is 0.6%, a dose much lower than the standard 1% inclusion rate.

| Table 1. Inactivation of ASFV (BA71V) at varying concentrations of MCFA in 20% DMSO. | | | | | | | | | | | | | | | | | | |
|---|---|---|---|---|---|---|---|---|---|---|---|---|---|---|---|---|---|---|
| | 2% | | | 1% | | | 0.5% | | | 0.25% | | | 0.125% | | | Pos. ctrl | | |
| undil. | - | - | - | - | - | - | - | - | - | - | - | - | + | + | + | + | + | + |
| 10⁻¹ | - | - | - | - | - | - | + | + | + | + | + | + | + | + | + | + | + | + |
| 10⁻² | - | - | - | - | - | - | + | + | + | + | + | + | + | + | + | + | + | + |
| 10⁻³ | - | - | - | - | - | - | - | - | - | + | + | + | + | + | + | + | + | + |
| 10⁻⁴ | - | - | - | - | - | - | - | - | - | - | - | - | + | + | + | + | + | + |
| 10⁻⁵ | - | - | - | - | - | - | - | - | - | - | - | - | - | - | - | - | + | + |
| 10⁻⁶ | - | - | - | - | - | - | - | - | - | - | - | - | - | - | - | - | - | - |
| 10⁻⁷ | - | - | - | - | - | - | - | - | - | - | - | - | - | - | - | - | - | - |
| TCID₅₀/ml | | - | | | - | | 2.53x10³=10^{3.4} | | | 2.53x10⁴=10^{4.4} | | | 2.53x10⁵=10^{5.4} | | | 1.42x10⁶=10^{6.15} | | |
| Log decrease | | - | | | - | | 2.75 | | | 1.75 | | | 0.75 | | | - | | |

| | 0.9% | | | 0.8% | | | 0.7% | | | 0.6% | | | 0.5% | | | Pos. ctrl | | |
|---|---|---|---|---|---|---|---|---|---|---|---|---|---|---|---|---|---|---|
| undil. | - | - | - | - | - | - | - | - | - | - | - | - | - | - | - | + | + | + |
| 10⁻¹ | - | - | - | - | - | - | - | - | - | + | + | + | + | + | + | + | + | + |
| 10⁻² | - | - | - | - | - | - | - | - | - | - | - | - | - | + | + | + | + | + |
| 10⁻³ | - | - | - | - | - | - | - | - | - | - | - | - | - | - | - | + | + | + |
| 10⁻⁴ | - | - | - | - | - | - | - | - | - | - | - | - | - | - | - | + | + | + |
| 10⁻⁵ | - | - | - | - | - | - | - | - | - | - | - | - | - | - | - | - | + | + |
| 10⁻⁶ | - | - | - | - | - | - | - | - | - | - | - | - | - | - | - | - | - | - |
| 10⁻⁷ | - | - | - | - | - | - | - | - | - | - | - | - | - | - | - | - | - | - |
| TCID₅₀/ml | | - | | | - | | | - | | 2.53x10²=10^{2.4} | | | 2.53x10³=10^{3.4} | | | 1.42x10⁶=10^{6.15} | | |
| Log decrease | | - | | | - | | | - | | 3.75 | | | 3 | | | - | | |
| *Data is shown as positive (+) or negative (-) for ASFV on virus isolation. Virus titration was performed on vero cells in triplicate using a monoclonal Ab against ASFV p30. | | | | | | | | | | | | | | | | | | |

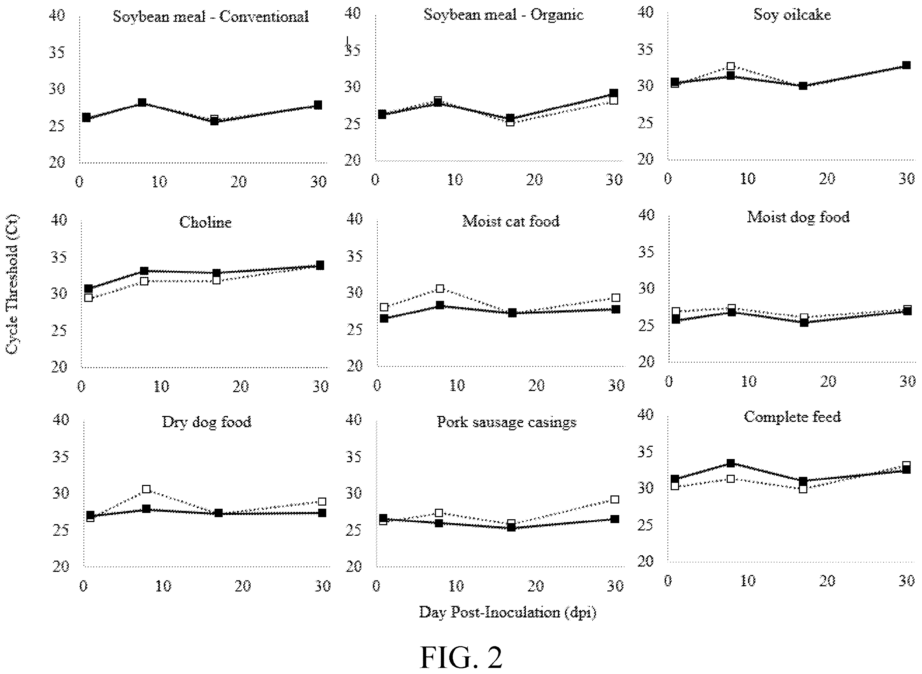

A 1% (weight %) medium chain fatty acids inclusion rate in 9 high-risk ingredients were also tested for ASFV survival using the ASFV Georgia 2007 isolate in a 30 transboundary model that simulates varying environmental temperature and humidity conditions. ASFV Georgia 2007 is the highly virulent ASFV isolate currently circulating in China. The high-risk feed ingredients include soybean meal conventional, soybean meal organic, soy oilcake, choline, moist cat food, moist dog food, dry dog food, pork sausage casings, and complete feed. Detection and quantification of ASFV DNA was performed by qPCR and compared between untreated inoculated feed and medium chain fatty acid-treated inoculated feed. In the first study, feed was treated with a 1% medium chain fatty acids inclusion of C6:C8:C10 at a 1:1:1 ratio at 0 days post-inoculation (dpi) immediately prior to ASFV inoculation. The PCR results demonstrated that all untreated control samples and 0 dpi MCFA-treated samples were positive for ASFV DNA on days 1, 8, 17 and 30 (Fig. 2). In Fig. 2, data is shown for untreated controls (open boxes) and samples treated with MCFA immediately prior to ASFV-inoculation on 0 dpi (black boxes). All samples had detectable ASFV DNA at the conclusion of the 30 day transboundary model. Ct values >40 were considered negative. Note: PCR detects virus DNA but does not test its infectivity.

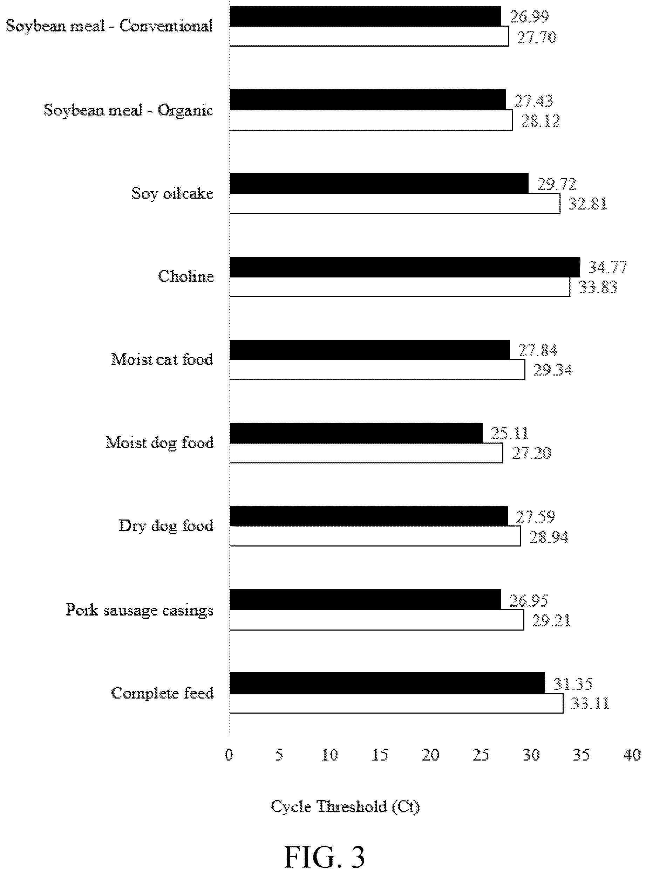

In the second study, detection and quantification of ASFV DNA was compared between untreated inoculated feed and medium chain fatty acid-treated inoculated feed which had been treated with the 1:1:1 1% medium chain fatty acids at 28 dpi. The results demonstrated that all untreated and 28 dpi medium chain fatty acid-treated samples were positive for ASFV DNA at 30 dpi (Fig. 3). In Fig. 3, data is shown as the mean cycle threshold (Ct) of duplicate replicates. All samples were positive for ASFV DNA at the conclusion of the transboundary model. Ct values >40 were considered negative.

Untreated inoculated feed and inoculated feed treated with 1% medium chain fatty acids at both 0 dpi and 28 dpi were then tested by virus isolation on porcine alveolar macrophages to determine if the ASFV DNA detected by PCR was infectious. Virus isolation determined that infectious virus was present in all untreated positive controls, whereas infectious virus was not detectable in any samples treated with medium chain fatty acids at either 0 dpi or 28 dpi (Table 2). Infectious virus was detected in positive untreated samples using a monoclonal antibody against the ASFV p30 protein.

| Table 2. Detection of ASFV Georgia 2007 by virus isolation at the conclusion of the 30 day transboundary model in feed and feed ingredients exposed to MCFA at 0 days post-inoculation (dpi) or 28 dpi* | | | | |
|---|---|---|---|---|
| Sample | Feed Ingredients | No MCFA Treatment† | MCFA at 0 dpi | MCFA at 28 dpi |
| 1 | Soybean meal - Conventional | + (10^{3.0}) | - | - |
| 2 | Soybean meal - Organic | + (10^{3.0}) | - | - |
| 3 | Soy oilcake | + (10^{3.1}) | - | - |
| 6 | Choline | + (10^{3.2}) | - | - |
| 8 | Moist cat food | + (10^{3.0}) | - | - |
| 9 | Moist dog food | + (10^{2.8}) | - | - |
| 10 | Dry dog food | + (10^{2.7}) | - | - |
| 11 | Pork sausage casings | + (10^{2.9}) | - | - |
| 12 | Positive control complete feed | + (10^{2.7}) | - | - |
| 13 | Negative control complete feed | - | ND | ND |
| *Data is shown as positive (+) or negative (-) for ASFV on virus isolation at 30 dpi. Virus isolation was performed on porcine alveolar macrophages in triplicate using a monoclonal Ab against ASFV p30. ND, not determined. | | | | |
| †Titers are shown as mean TCID₅₀ in positive controls; Initial virus inoculation was 10⁵ TCID₅₀ | | | | |

Samples treated with medium chain fatty acids at either 0 dpi or 28 dpi were then further tested in a nursery pig bioassay model to assess for the presence of infectious virus. Samples from medium chain fatty acid-treated feed were injected intramuscularly as this is the most sensitive method to detect infectious ASFV. Pigs were injected with either 1 or 2 samples to reduce the number of pigs utilized. Pooled samples were based on quantitative PCR results. All feed samples treated with medium chain fatty acids at 0 dpi were negative for infectious ASFV on pig bioassay (Table 3). All but 2 feed samples treated with medium chain fatty acids at 28 dpi were negative for infectious ASFV on pig bioassay (Table 3). The two feed samples (of which one or both may have had infectious ASFV present) were soybean meal organic and dry dog food. These two samples were injected into a single pig which had ASFV detected on virus isolation of spleen. Overall, our data support medium chain fatty acids being an effective mitigant for infectious ASFV in cell culture and in feed ingredients.

| Table 3. Detection of ASFV Georgia 2007 by pig bioassay at the conclusion of the 30 day transboundary model in feed and feed ingredients exposed to MCFA at 0 days post-inoculation (dpi) or 28 dpi* | | | | | |
|---|---|---|---|---|---|
| Pig Number | Pooled Samples† | MCFA at 0 dpi | Pig Number | Pooled Samples† | MCFA at 28 dpi |
| 817 | 11 | - | 344 | 9 | - |
| 844 | 9, 10 | - | 343 | 1, 11 | - |
| 811 | 1, 8 | - | 346 | 2, 10 | + |
| 845 | 2, 12 | - | 342 | 3, 8 | - |
| 800 | 3, 6 | - | 459 | 6, 12 | - |
| 854 | 13 | - | 337 | 13 | - |
| *Data is shown as positive (+) or negative (-) bioassay results for ASFV after intramuscular injection of feed supernatant collected at 30 dpi and tested in nursery pigs. No more than 2 samples were tested in each pig. Samples were pooled based on PCR values from 30 dpi. Positive and negative results were determined based on ASFV PCR of serum and spleen, and virus isolation on spleen from inoculated pigs. Samples were considered positive for the presence of infectious ASFV if one or more of the diagnostic tests were positive. | | | | | |
| †Key: 1, Soybean meal - Conventional; 2, Soybean meal - Organic; 3, Soy oilcake; 6, Choline; 8, Moist cat food; 9, Moist dog food; 10, Dry dog food; 11, Pork sausage casings; 12, Positive control complete feed; 13, Negative control complete feed | | | | | |

### EXAMPLE II

### Classical swine fever virus testing

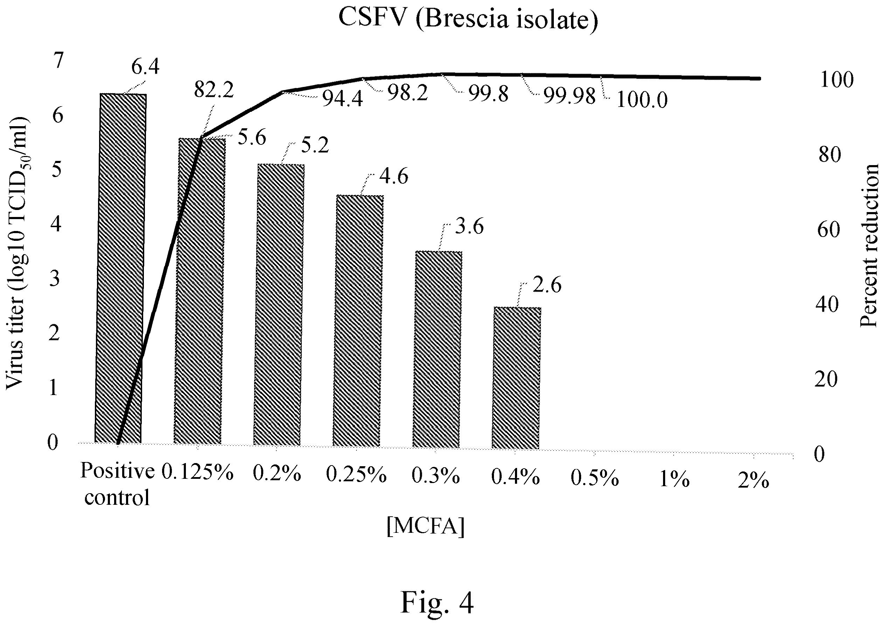

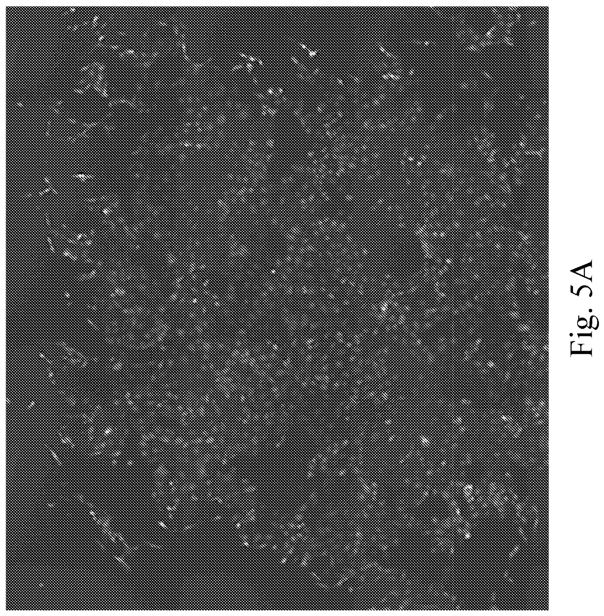

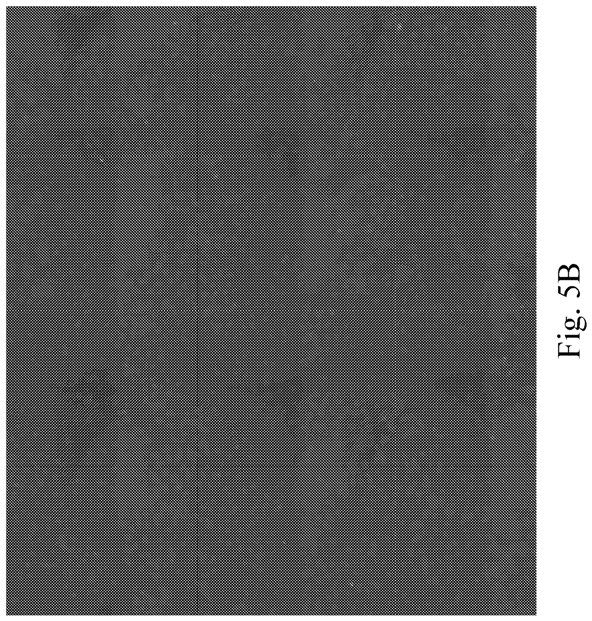

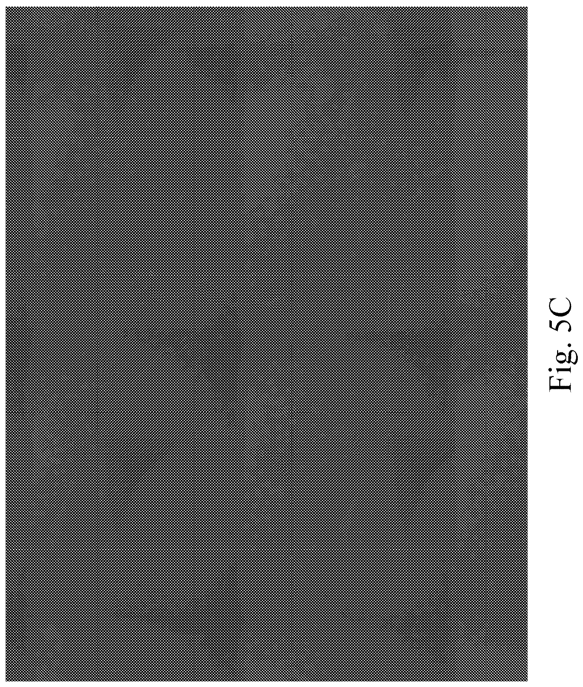

The effects of MCFA on CSFV in cell culture are shown in Table 4, as well as Figs. 4 and Figs. 5A-5C. Various levels of the medium chain fatty acid treatment (1:1:1 ratio of C6:C8:C10) were tested for efficacy in inactivating or reducing viral titers of CSFV Brescia isolate in a cell culture model. MCFA levels tested between 10% and 0.5% reduced the CSF viral titers to levels below what is detectable by indirect fluorescent antibody testing on porcine kidney cells. As shown in Fig. 5B, no CSFV was detectable after MCFA treatment. Dose-dependent reductions in CSFV viral titers were shown after exposure to MCFA at levels between 0.4% and 0.125%. The lowest MCFA inclusion rate tested (0.125%) resulted in an 82.2% reduction in viral titer when compared to the non-mitigated positive control.

| Table 4. Inactivation of CSFV (Brescia isolate) at varying concentrations of MCFA in 20% DMSO in a cell culture model. | | | | | | | | | | | | | | | | | | |
|---|---|---|---|---|---|---|---|---|---|---|---|---|---|---|---|---|---|---|
| | 2% | | | 1% | | | 0.5% | | | 0.25% | | | 0.125% | | | Pos. ctrl | | |
| undil. | - | - | - | - | - | - | - | - | - | - | - | - | - | - | - | + | + | + |
| 10⁻¹ | - | - | - | - | - | - | - | - | - | + | + | + | + | + | + | + | + | + |
| 10⁻² | - | - | - | - | - | - | - | - | - | + | + | + | + | + | + | + | + | + |
| 10⁻³ | - | - | - | - | - | - | - | - | - | + | + | + | + | + | + | + | + | + |
| 10⁻⁴ | - | - | - | - | - | - | - | - | - | - | - | + | + | + | + | + | + | + |
| 10⁻⁵ | - | - | - | - | - | - | - | - | - | - | - | - | - | - | + | + | + | + |
| 10⁻⁶ | - | - | - | - | - | - | - | - | - | - | - | - | - | - | - | - | - | - |
| 10⁻⁷ | - | - | - | - | - | - | - | - | - | - | - | - | - | - | - | - | - | - |
| TCID₅₀/ml | | - | | | - | | | - | | 4.5x10⁴=10^{4.6} | | | 4.5x10⁵=10^{5.6} | | | 2.53x10⁶=10^{6.4} | | |
| Log decrease | | - | | | - | | | - | | 1.8 | | | 0.8 | | | - | | |

| | 0.5% | | | 0.4% | | | 0.3% | | | 0.2% | | |
|---|---|---|---|---|---|---|---|---|---|---|---|---|
| undil. | | | | - | - | - | - | - | - | - | - | - |
| 10⁻¹ | | | | + | + | + | + | + | + | + | + | + |
| 10⁻² | | | | - | + | - | + | + | + | + | + | + |
| 10⁻³ | | | | - | - | - | - | - | + | + | + | + |
| 10⁻⁴ | | | | - | - | - | - | - | - | - | + | + |
| 10⁻⁵ | | | | - | - | - | - | - | - | - | - | - |
| 10⁻⁶ | | | | - | - | - | - | - | - | - | - | - |
| 10⁻⁷ | | | | - | - | - | - | - | - | - | - | - |
| TCID₅₀/ml | | | | 4.5x10²=10^{2.6} | | | 4.5x10³=10^{3.6} | | | 1.42x10⁵=10^{5.15} | | |
| Log decrease | | | | 3.8 | | | 2.8 | | | 1.25 | | |
| *Data is shown as positive (+) or negative (-) for CSFV on virus titration on porcine kidney cells. Titrations were performed in triplicate and virus was detected by IFA using a PrioMab CSFV V3 monoclonal antibody. | | | | | | | | | | | | |

The effects of MCFA on CSFV survival in feed ingredients subjected to transboundary environmental conditions were also tested. A 1% MCFA blend (1:1:1 ratio of C6:C8:C10) was tested for its ability to inactivate CSFV in two feed ingredients which supported CSFV survival over a 37-day transboundary model simulating shipment. The two ingredients tested included soybean meal conventional and pork sausage casings. The MCFA blend was added to the feed ingredient and vortexed to mix MCFA throughout the feed ingredient prior to CSFV inoculation (10⁵ TCID₅₀) at 0 days post-inoculation (dpi). As early as 1 dpi, samples treated with MCFA were negative on virus isolation and titration whereas positive controls had detectable CSFV titers of 10^{4.3} TCID₅₀ and 10^{3.7} TCID₅₀, for soybean meal conventional and pork sausage casings, respectively. By 37 dpi, all samples (including the untreated positive controls) were negative on virus isolation and titration. Supernatant from conventional soybean meal samples with and without MCFA treatment were collected at the conclusion of the 37-day transboundary model and tested in a 3-week-old pig bioassay by intramuscular injection. Untreated soybean meal samples were CSFV positive on pig bioassay whereas soybean meal samples treated with MCFA were negative for CSFV on pig bioassay, demonstrating the efficacy of MCFA in eliminating CSFV infectivity in soybean meal subjected to conditions of transboundary shipment.

The present invention will now be described by the following non-limiting sequentially numbered statements:
1. A method of inhibiting African swine fever virus and/or classical swine fever virus in animal feed or animal feed ingredients, said method comprising:
   introducing a chemical mitigant to said feed or feed ingredients, said chemical mitigant comprising a medium chain fatty acid and/or an essential oil,
   wherein said chemical mitigant is introduced at an inclusion rate of from about 0.125 weight % to less than 2 weight %, based upon the total weight of the animal feed or feed ingredient taken as 100% by weight.
2. The method of statement 1, wherein said medium chain fatty acid is selected from the group consisting of caproic acid, caprylic acid, capric acid, lauric acid, and mixtures thereof.
3. The method of statement 1 or 2, wherein said chemical mitigant is a blend of two or more medium chain fatty acids.
4. The method of any one of statements 1 to 3, wherein said chemical mitigant comprises a blend of medium chain fatty acids comprising caproic acid, caprylic acid, and capric acid.
5. The method of any one of statements 1 to 4, wherein said chemical mitigant comprises a blend of medium chain fatty acids comprising approximately equal portions of caproic acid, caprylic acid, and capric acid.
6. The method of any one of statements 1 to 5, wherein said essential oil is selected from the group consisting of garlic oleoresin, turmeric oleoresin, capsicum oleoresin, rosemary extract, wild oregano essential oil, and mixtures thereof.
7. The method of any one of statements 1 to 6, wherein said chemical mitigant comprises a blend of medium chain fatty acids and essential oils.
8. The method of any one of statements 1 to 7, wherein said animal feed or animal feed ingredients is selected from the group consisting of complete swine diet, blood meal, porcine meat and bone meal (MBM), spray-dried animal plasma, feather meal, avian blood meal, poultry by-product meal, vitamin D, lysine hydrochloride, choline chloride, and soybean meal.
9. The method of any one of statements 1 to 7, wherein said animal feed or animal feed ingredients is dry pet kibble, said method further comprising applying sodium bisulfate to the surface of said dry pet kibble.
10. The method of statement9, wherein said sodium bisulfate is applied to the surface of said dry pet kibble at an inclusion rate of from about 0.1 weight % to about 2 weight %, based upon the total weight of the animal feed or feed ingredients taken as 100% by weight.
11. A chemical mitigant for use in inhibiting African swine fever virus and/or classical swine fever virus in animal feed or animal feed ingredients, said chemical mitigant comprising a medium chain fatty acid and/or an essential oil.
12. The chemical mitigant of statement11, wherein said medium chain fatty acid is selected from the group consisting of caproic acid, caprylic acid, capric acid, lauric acid, and mixtures thereof.
13. The chemical mitigant of statement11 or 12, wherein said chemical mitigant is a blend of two or more medium chain fatty acids.
14. The chemical mitigant of any one of statements 11 to 13, wherein said chemical mitigant comprises a blend of medium chain fatty acids comprising caproic acid, caprylic acid, and capric acid.
15. The chemical mitigant of any one of statements 11 to 14, wherein said chemical mitigant comprises a blend of medium chain fatty acids comprising approximately equal portions of caproic acid, caprylic acid, and capric acid.
16. The chemical mitigant of any one of statements 11 to 15, wherein said essential oil is selected from the group consisting of garlic oleoresin, turmeric oleoresin, capsicum oleoresin, rosemary extract, wild oregano essential oil, and mixtures thereof.
17. The chemical mitigant of any one of statements 11 to 16, wherein said chemical mitigant comprises a blend of medium chain fatty acids and essential oils.
18. The chemical mitigant of any one of statements 11 to 17, wherein said chemical mitigant comprises a blend of essential oils comprising approximately equal portions of garlic oleoresin, turmeric oleoresin, capsicum oleoresin, rosemary extract, and wild oregano essential oils.
19. A treated animal feed or animal feed ingredient having resistance to African swine fever virus and/or classical swine fever virus, comprising from about 0.125 weight % to less than 2 weight % of a chemical mitigant, based upon the total weight of the animal feed or feed ingredient taken as 100% by weight, said chemical mitigant comprising a medium chain fatty acid and/or an essential oil, wherein said animal feed or animal feed ingredient is selected from the group consisting of complete swine diet, blood meal, porcine meat and bone meal (MBM), spray-dried animal plasma, feather meal, avian blood meal, poultry by-product meal, vitamin D, lysine hydrochloride, choline chloride, soybean meal, dry pet kibble, and mixtures thereof.
20. A treated animal feed or animal feed ingredient having resistance to African swine fever virus and/or classical swine fever virus according to statement19, wherein said animal feed or animal feed ingredients is dry pet kibble, further comprising sodium bisulfate of the surface of said dry pet kibble.

## Claims

1. A method of inhibiting classical swine fever virus in animal feed or animal feed ingredients, said method comprising:
introducing a chemical mitigant to said feed or feed ingredients, said chemical mitigant comprising a medium chain fatty acid and/or an essential oil,
wherein said chemical mitigant is introduced at an inclusion rate of from about 0.125 weight % to less than 2 weight %, based upon the total weight of the animal feed or feed ingredient taken as 100% by weight.

2. The method of claim 1, wherein said medium chain fatty acid is selected from the group consisting of caproic acid, caprylic acid, capric acid, lauric acid, and mixtures thereof.

3. The method of claim 1 or 2, wherein said chemical mitigant
(i) is a blend of two or more medium chain fatty acids; or
(ii) comprises a blend of medium chain fatty acids and essential oils.

4. The method of any one of claims 1 to 3, wherein said chemical mitigant comprises a blend of medium chain fatty acids comprising caproic acid, caprylic acid, and capric acid;
preferably approximately equal portions of caproic acid, caprylic acid, and capric acid.

5. The method of any one of claims 1 to 4, wherein said essential oil is selected from the group consisting of garlic oleoresin, turmeric oleoresin, capsicum oleoresin, rosemary extract, wild oregano essential oil, and mixtures thereof.

6. The method of any one of claims 1 to 5, wherein said animal feed or animal feed ingredients is selected from the group consisting of complete swine diet, blood meal, porcine meat and bone meal (MBM), spray-dried animal plasma, feather meal, avian blood meal, poultry by-product meal, vitamin D, lysine hydrochloride, choline chloride, and soybean meal.

7. The method of any one of claims 1 to 5, wherein said animal feed or animal feed ingredients is dry pet kibble, said method further comprising applying sodium bisulfate to the surface of said dry pet kibble;
optionally wherein said sodium bisulfate is applied to the surface of said dry pet kibble at an inclusion rate of from about 0.1 weight % to about 2 weight %, based upon the total weight of the animal feed or feed ingredients taken as 100% by weight.

8. A chemical mitigant for use in inhibiting classical swine fever virus in animal feed or animal feed ingredients, said chemical mitigant comprising a medium chain fatty acid and/or an essential oil.

9. The chemical mitigant of claim 8, wherein said medium chain fatty acid is selected from the group consisting of caproic acid, caprylic acid, capric acid, lauric acid, and mixtures thereof.

10. The chemical mitigant of claim 8 or 9, wherein said chemical mitigant is
(i) a blend of two or more medium chain fatty acids; or
(ii) comprises a blend of medium chain fatty acids and essential oils.

11. The chemical mitigant of any one of claims 8 to 10, wherein said chemical mitigant comprises a blend of medium chain fatty acids comprising caproic acid, caprylic acid, and capric acid;
preferably approximately equal portions of caproic acid, caprylic acid, and capric acid.

12. The chemical mitigant of any one of claims 8 to 11, wherein said essential oil is selected from the group consisting of garlic oleoresin, turmeric oleoresin, capsicum oleoresin, rosemary extract, wild oregano essential oil, and mixtures thereof.

13. The chemical mitigant of any one of claims 8 to 12, wherein said chemical mitigant comprises a blend of essential oils comprising approximately equal portions of garlic oleoresin, turmeric oleoresin, capsicum oleoresin, rosemary extract, and wild oregano essential oils.

14. A treated animal feed or animal feed ingredient having resistance to classical swine fever virus, comprising from about 0.125 weight % to less than 2 weight % of a chemical mitigant, based upon the total weight of the animal feed or feed ingredient taken as 100% by weight, said chemical mitigant comprising a medium chain fatty acid and/or an essential oil, wherein said animal feed or animal feed ingredient is selected from the group consisting of complete swine diet, blood meal, porcine meat and bone meal (MBM), spray-dried animal plasma, feather meal, avian blood meal, poultry by-product meal, vitamin D, lysine hydrochloride, choline chloride, soybean meal, dry pet kibble, and mixtures thereof.

15. A treated animal feed or animal feed ingredient having resistance to classical swine fever virus according to claim 15, wherein said animal feed or animal feed ingredients is dry pet kibble, further comprising sodium bisulfate of the surface of said dry pet kibble.
